(19) Europäisches Patentamt
European Patent Office
Office européen des brevets

(11) Publication number: 0 183 797 B1

(12) **EUROPEAN PATENT SPECIFICATION**

(45) Date of publication of patent specification: 19.07.89

(51) Int. Cl.[4]: C 07 D 319/06, C 07 D 319/08, C 07 C 67/313

(21) Application number: 85902887.0

(22) Date of filing: 30.05.85

(86) International application number: PCT/US85/00987

(87) International publication number: WO 86/00068 03.01.86 Gazette 86/01

(54) **5-HALO-4H-1,3-DIOXIN-4-ONE COMPOUNDS, PROCESS FOR THEIR PREPARATION AND THEIR USE IN THE PREPARATION OF ALPHA-HALO-ACETOACETIC ESTERS.**

(30) Priority: 11.06.84 US 619449
11.06.84 US 619450

(43) Date of publication of application: 11.06.86 Bulletin 86/24

(45) Publication of the grant of the patent: 19.07.89 Bulletin 89/29

(84) Designated Contracting States: CH DE FR GB LI

(56) References cited:
Journal of organic chemistry, vol. 47, 1982, pp. 2823-4 (Washington, US), R. Boeckman et al.: "Methodology for the synthesis of phosphorus - activated tetramic acids: Application to the synthesis of unsaturated 3-acetyl-tetramic acids"

(73) Proprietor: EASTMAN KODAK COMPANY (a New Jersey corporation)
343 State Street
Rochester New York 14650 (US)

(72) Inventor: CLEMENS, Robert, Jay
113 Olympus Drive
Kingsport, TN 37663 (US)

(74) Representative: Parent, Yves et al
Kodak-Pathé Département Brevets et Licences
Centre de Recherches et de Technologie Zone Industrielle
F-71102 Chalon-sur-Saône Cédex (FR)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 183 797 B1

**Description**

BACKGROUND OF THE INVENTION

This invention relates to halogenated 4H-1,3-dioxin-4-one compounds and their preparation and the use of such halogenated 4H-1,3-dioxin-4-ones for the preparation of α-haloacetoacetic esters.

The preparation of acetoacetic esters by the reaction of alcohols and diketene in the presence of an acid catalyst has been described by A. B. Boese in *Ind. Eng. Chem.* 32, 16 (1940). The sodium enolate salt of ethyl acetoacetate has been reported by Chick et al, *Journal of American Chemical Society* 93, 946 (1908) and 97, 1978 (1910) to be produced by reacting diketene and sodium ethoxide in dry alcohol. The desired α-halogenated product may be obtained by treating the acetoacetic ester with a suitable halogenating agent, e.g. $SO_2Cl_2$. [See Boehme, *Org. Syn. Coll.,* Vol. 4, 590 (1963)].

Reported by Blomquist et al in *Journal of American Chemical Society,* Vol. 70, page 29 (1948) is the preparation of ethyl 2-bromoacetoacetate by first treating ketene dimer in chloroform solution with N-bromosuccinimide followed by reaction with ethyl alcohol. The 2-chloro derivative was similarly prepared by treating ketene dimer with N,2,4-trichloroacetanilide followed by reaction with ethyl alcohol. Reported yields are 43% and 35%, respectively.

A new intermediate compound, halogenated 4H-1,3-dioxin-4-one, has now been found which provides an alternate method for producing α-haloacetoacetic esters.

It has been found that by reacting a halogenating agent (i.e., $X_2$ or $SO_2X_2$ wherein X is Cl or Br) with a 4H-1,3-dioxin-4-one compound the monohalogenated derivative, 5-halo-4H-1,3-dioxin-4-one, is obtained.

The 5-halo-4H-1,3-dioxin-4-ones are obtained in a relatively short reaction time and excellent yield. The unpurified product, for many uses, need not be purified before being used in subsequent reactions. In addition, halogenation is achieved without the use of expensive halogenating agents. These 5-halo-4H-1,3-dioxin-4-ones can then be reacted with an alkali metal alkoxide or aralkoxide or with a phenol compound to give acetoacetic esters monohalogenated at the α-position or their salts, respectively.

The α-haloacetoacetic esters obtained by this process are useful intermediates for synthesizing a wide variety of compounds including, for example, pharmaceuticals and dye intermediates as described in U.S. Patent No. 3,876,647 (1975); *J. Am. Chem. Soc.* 57, 1876 (1935); *J. Org. Chem.* 43, 3821 (1978); and *Chem. Ber.* 88, 130 (1955).

SUMMARY OF THE INVENTION

The present invention relates to 5-halo-4H-1,3-dioxin-4-one compounds, α-haloacetoacetic esters prepared from such compounds and to processes for the preparation of such compounds. The 5-halo-4H-1,3-dioxin-4-one compounds have the formula

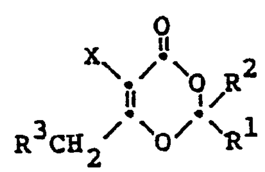

(II)

and are produced by treating a compound having the formula

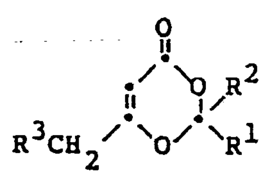

(I)

with $X_2$ or $SO_2X_2$ and recovering the product resulting therefrom.

The 5-halo-4H-1,3-dioxin-4-ones are then reacted with an alkali metal alkoxide at a temperature of about −40°C to about 50°C to give an α-haloacetoacetic ester. This reaction may be represented by the following reaction

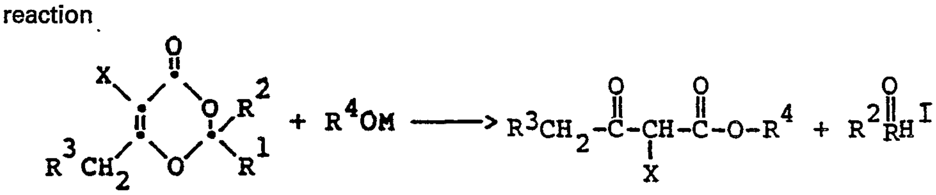

(II) (IV) (III)

In the above formulae X is Cl or Br; $R^1$ and $R^2$ are each independently alkyl, aryl, substituted aryl, or collectively alkylene; $R^3$ is hydrogen, Cl, Br, an aliphatic hydrocarbon residue, alkoxy, aryl, substituted aryl, or a hetero substituent. $R^4$ is alkyl, aralkyl or aryl; and M is an alkali metal atom if $R^4$ denotes alkyl or aralkyl,

or M is hydrogen if $R^4$ denotes aryl. If M is an alkali metal atom, the product of the formula III is obtained in the form of the alkali metal salt of its enolic tautomer.

DETAILED DESCRIPTION OF THE INVENTION

The invention described herein relates to 5-halo-4H-1,3-dioxin-4-one compounds, α-haloacetoacetic esters prepared from such compounds and processes for their preparation.

$R^1$ and $R^2$ of the above formulae, are residues of the aliphatic or cycloaliphatic ketone from which the 4H-1,3-dioxin-4-one compounds are derived. The alkyl substituents for $R^1$ and $R^2$ generally can be lower alkyl (i.e., $C_1$—$C_6$), either branched or straight chain.

Examples of these include methyl, ethyl, propyl and isobutyl. The aryl substituents for $R^1$ and $R^2$ generally can be phenyl and the substituted aryl group may contain any substituent which is not reactive with the halogenating agent or alkali metal alkoxide or does not otherwise interfere with the course of the reactions. Examples of substituted aryl groups include p-nitro- phenyl and o-chlorophenyl. Examples of the alkylene groups are tetramethylene and pentamethylene [i.e., $—CH_2(CH_2)_2CH_2—$ and $—CH_2(CH_2)_3CH_2—$]. Most commonly and preferably $R^1$ and $R^2$ are each methyl.

The aliphatic hydrocarbon substituent for $R^3$ of the above formulae may be saturated or unsaturated and straight or branched and generally comprises about $C_1$—$C_{20}$ carbon atoms. The alkoxy substituent generally can have 1—4 carbon atoms. Examples of the alkoxy substituent include methoxy and ethoxy. The aryl substituent generally is intended to mean phenyl or substituted phenyl and includes, for example, p-nitrophenyl. Examples of the hetero substituents include phenylthio, anilino and diethyl phosphonato [i.e., $(CH_3CH_2)_2PO$].

The alkyl substituent and the alkyl moiety of the aralkyl substituent for $R^4$ are generally lower alkyl (i.e., $C_1$—$C_6$). Examples of alkyl substituents include methyl, ethyl, isopropyl, isobutyl, and examples of aralkyl substituents include benzyl. Preferably, $R^4$ will be methyl or ethyl. The aryl substituent for $R^4$ includes phenyl. The compounds of the formula IV wherein $R^4$ denotes aryl and M denotes hydrogen are also referred to as phenol compounds.

The alkali metal ion, represented by M in the above formula if $R^4$ denotes alkyl or aralkyl, may be sodium, lithium or potassium with sodium being preferred for reasons of economy.

The starting materials as shown by formula (I) are known in the art and/or are readily obtained by methods known to one skilled in the art. [For example, see Boeckman, *Journ. Org. Chem.* 47, 2823—2824 (1982); Blomquist, *Journ, Amer. Chem. Soc.* 70, 29—30 (1948)].

The 5-halo compounds of formula II are prepared by treating a 1,3-dioxin-4-one of the formula

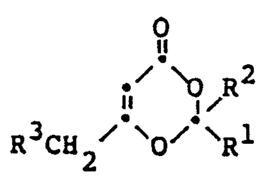

(I)

with $X_2$ or $SO_2X_2$, wherein X is Cl or Br.

Elemental chlorine or bromine are the preferred halogenating agents. The conditions of the reaction may be varied considerably, depending on the halogenating agent used and whether the reaction is carried out in a gaseous or liquid phase. In carrying out the reaction, for example, using elemental chlorine or bromine and in a liquid phase the reaction is carried out at temperatures and pressure sufficient to maintain chlorine or bromine in liquid form, i.e. at atmospheric pressure the temperature for chlorine will be below −35°C and below 59°C for bromine. Of course, higher temperatures may be used with the use of elevated pressures. It was unexpected that halogenation in this manner would give monohalogenation at the 5-position of the dioxinone ring.

In carrying out the reaction in a liquid phase a solvent is not required but one may be used if desired. Typical solvents which may be used are, for example, aliphatic or aromatic hydrocarbons, or chlorinated aliphatic or aromatic hydrocarbons, including, for example, methylene chloride, chloroform, benzene, chlorobenzene and the like. The amount of solvent generally will be dictated by economy and convenience. A by-product of the halogenation reaction is a hydrogen halide. This by-product generally may be removed by addition of a base to the product mixture such as an amine or carbonate salt, including, for example, pyridine triethylamine, or sodium carbonate.

The halogenating agent will generally be employed in stoichiometric amounts and preferably in slight excess, up to about 1.2 moles per mole of (I). Advantageously, monohalogenation of (I) is achieved rapidly, giving 5-halo-4H-1,3-dioxin-4-ones in excellent yield and of sufficient purity as not to require purification prior to the use thereof in subsequent reactions. Of course, when $R^3$ is Cl or Br the dihalogenated compound will be the resulting product. When a product of even higher purity is desired the crude 5-halo-4H-1,3-dioxin-4-ones may be purified, for example, by column chromatography or distillation with wiped-film evaporation.

The 5-halo-4H-1,3-dioxin-4-ones (II) can then be used in the preparation of α-haloacetoacetic esters (III). Depending on the acetoacetic ester desired, these compounds may be reacted with a phenol compound or with an alkali metal alkoxide or aralkoxide (IV) to obtain α-haloacetoacetic esters of the formula

$$R^3\text{-}CH_2\text{-}CO\text{-}\underset{\underset{X}{|}}{CH}\text{-}CO\text{-}O\text{-}R^4 \qquad (III)$$

or the alkali metal salt of the corresponding enolic tautomers, respectively.

In the above formula X is Cl or Br, $R^4$ is the residue of the phenol compound or of the alkali metal alkoxide or aralkoxide reactant as previously defined, and $R^3$ is as previously defined.

The mole ratio of alkali metal alkoxide, aralkoxide or phenol reactant (IV), to 5-halo-4H-1,3-dioxin-4-one (II) generally may be in the range of at least 1.0 up to about 2.0 with best results being obtained with a mole ratio of about 1.0 to about 1.2.

The temperatures at which the reaction can be carried out will depend on the particular acetoacetic ester prepared, i.e. depending in part on whether the reactant (IV) is a phenol compound or an alkali metal alkoxide or aralkoxide. Temperatures in the range of about −40° to about 50°C and preferably ambient temperatures, i.e. 0—25°C, will generally be used with an alkali metal alkoxide or aralkoxide. With a phenol compound, the reaction temperature may range from about 100° to about 200°C and preferably about 120° to about 145°C. Thus, the range of temperatures at which the α-haloacetoacetic esters can be prepared can vary from about −40°C up to about 200°C.

A solvent may be used in preparing the α-haloacetoacetic esters if desired. When an alkali metal alkoxide is used the solvent desirably is an alkanol having the same number of carbon atoms as the alkoxide reactant. When the reactant (IV) is a phenol compound the solvent can be, for example, toluene, xylene, and the like. The amount of solvent is not critical to the reaction and generally will be dictated by economy and convenience.

When the reactant (IV) is an alkali metal alkoxide or aralkoxide, the α-haloacetoacetic ester initially formed is an enolate salt which can be reacted further to produce other derivatives or converted to the free ester by acidification.

The following examples are given to further illustrate the invention, but it is to be understood that the invention is not to be limited in any way by the details described therein.

## Example 1

Preparation of 5-Bromo-2,2,6-Trimethyl-4H-1,3-Dioxin-4-One

Bromine (0.105 mol, 5.38 ml) was added dropwise to a 20°C solution of 2,2,6-trimethyl-4H-1,3-dioxin-4-one (TKD, 0.1 mol, 14.2 g) in 100 ml $CH_2Cl_2$ over five minutes. The reaction solution was rapidly decolorized and hydrogen bromide evolved. After 15 minutes, the solvent was removed *in vacuo* to provide 22 g (99%, >95% pure by NMR) of a pale yellow oil which solidified upon refrigeration. Flash chromatography (10% ether/hexane on silica) afforded 16.5 g (75%) of the title compound as colorless plates.

## Example 2

Preparation of 5-Chloro-2,2,6-Trimethyl-1,3-Dioxin-4-One

A solution of 2,2,6-trimethyl-1,3-dioxin-4-one (0.2 mol, 28.4 g) in 100 ml of $CH_2Cl_2$ was cooled to −50°C and liquid chlorine (0.25 mol) was added dropwise over five minutes. The solution was allowed to warm to 20°C over 30 minutes, and the solvent was then removed *in vacuo*, leaving 35.8 g (100% yield, 92% assay) of a pale yellow oil. A portion of this mixture was purified by flash chromatography (10% $Et_2O$/hexanes on silica) to provide the title compound as white crystals.

## Example 3

Preparation of 4-Nitrophenyl 2-Chloroacetoacetate

A solution of 5-chloro-2,2,6-trimethyl-4H-1,3-dioxin-4-one (8.8 g, 50 mmol) and 4-nitrophenol (7.65 g, 55 mmol) in 5 ml of xylene under continuous nitrogen purge was immersed in an oil bath preheated to 110°C. The reaction was then heated to 135°C and then stirred an additional 15 minutes. The pale brown reaction was cooled to 20°C and the precipitated product washed with ether/hexanes to afford 10.9 g (84%) of the title compound as flaky, white crystals.

## Example 4

Preparation of Methyl-α-Chloroacetoacetate

A solution of 25% sodium methoxide in methanol (5 ml, 21.6 mmol) was added to a solution of 5-chloro-2,2,6-trimethyl-4H-1,3-dioxin-4-one (3.24 g, 18.4 mmol) in 10 ml of methanol at 20°C. The resulting yellow solution was stirred for 10 minutes, acidified with 10% HCl, and extracted with ether. Evaporation of the ether provided 2.35 g (94% yield, NMR assay .90%) of a pale yellow oil, which was distilled to provide 1.8 g (66%) of pure title compound as a colorless liquid.

## Example 5

Preparation of Ethyl-α-Chloroacetoacetate

Sodium metal (0.3 g, 12 mmol) was added to 10 ml of ethanol, and then 5-chloro-2,2,6-trimethyl-4H-1,3-dioxin-4-one (1.76 g, 10 mmol) was added. The reaction was stirred one hour at 20°C, during which time the sodium dissolved. The reaction partitioned between ether and saturated ammonium chloride which

had been acidified to pH .2 with HCl. Evaporation of the ethereal layer, followed by distillation afforded 1.15 g (70%) of the title compound as a colorless liquid.

Example 6

Preparation of Ethyl-α-Bromoacetoacetate

Sodium metal (13.7 g, 0.59 mol) was dissolved in 500 ml of absolute ethanol, and the resulting ethoxide solution was cooled to 0°C. Crude 5-bromo-2,2,6-trimethyl-4H-1,3-dioxin-4-one (115.5 g, 0.5 mole at 95%) purity) was then added, dropwise, to the chilled ethoxide solution over 30 minutes and the dark orange reaction mixture was stirred an additional 30 minutes at 0°C. The reaction was poured into 500 ml of ether and 550 ml of 1*N* HCl, and the organic layer was washed repeatedly with water (4 × 250 ml) and then dried over $Na_2SO_4$. Removal of solvent *in vacuo* (30°C, 2 Torr), followed by distillation on a 2-inch wiped-film molecular still (120°C jacket, 0.2 Torr) afforded 68.7 g (71%) of a pale yellow oil (98% pure by gc).

**Claims**

1. A compound having the formula

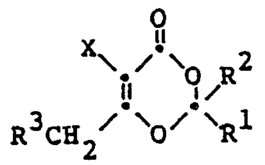

(II)

wherein X is Cl or Br; $R^1$ and $R^2$ are each independently alkyl, aryl, substituted aryl or collectively alkylene; and $R^3$ is hydrogen, Cl, Br, aliphatic hydrocarbyl, preferably of $C_1$—$C_{20}$, alkoxy wherein the alkyl moiety preferably is $C_1$—$C_4$, aryl, substituted aryl or a hetero substituent, preferably phenylthio, anilino, or diethylphosphonato.

2. The compound of Claim 1 wherein X is Cl or Br; $R^1$ and $R^2$ are each methyl or collectively tetramethylene or pentamethylene; and $R^3$ is hydrogen, Cl or Br.

3. The compound of Claim 1 wherein X is Cl or Br; $R^1$ and $R^2$ are each methyl; and $R^3$ is hydrogen.

4. A process for producing the compound of Claim 1 comprising treating a compound of the formula

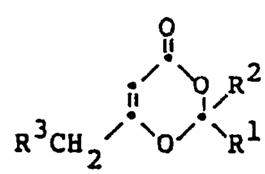

(I)

with $X_2$ or $SO_2X_2$ and recovering the 5-halo product resulting therefrom, wherein X, $R^1$, $R^2$ and $R^3$ are as defined in Claim 1.

5. The process according to Claim 4 wherein the mole ratio of $X_2$ or $SO_2X_2$ to compound (I) is about 1.0 to about 1.2.

6. The process according to Claim 4 comprising treating compound (I) with elemental chlorine or bromine and recovering the 5-halo product resulting therefrom.

7. The process according to Claim 6 wherein $R^1$ and $R^2$ are each methyl; and $R^3$ is hydrogen, Cl or Br.

8. A process for the preparation of an α-haloacetoacetic ester of the formula

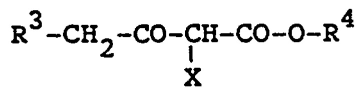

(III)

or of an alkali metal salt of its enolic tautomer, wherein X is Cl or Br; $R^3$ is hydrogen, Cl, Br, aliphatic hydrocarbyl, alkoxy, aryl, substituted aryl or a hetero substituent; and $R^4$ is alkyl, aralkyl or aryl; which comprises reacting a compound of the formula II with a reactant of the formula

$$R^4OM \qquad (IV)$$

wherein $R^4$ is alkyl, aralkyl or aryl and M is an alkali metal atom when $R^4$ is alkyl or aralkyl or M is hydrogen when $R^4$ is aryl.

9. A process according to Claim 8 for the preparation of an alkali metal salt of an α-haloacetoacetic ester comprising reacting a compound of formula II with an alkali metal alkoxide or aralkoxide of formula IV wherein $R^4$ is alkyl or aralkyl and M is an alkali metal atom at a temperature of about −40° to about 50°C.

10. The process of Claim 9 wherein the alkali metal alkoxide is sodium methoxide or sodium ethoxide.

11. The process of Claim 9 wherein the mole ratio of the alkali metal alkoxide to 5-halo-4H-1,3-dioxin-4-one compound is about 1.0 to about 2.0.

12. The process of Claim 9 comprising reacting an alkali metal alkoxide with 5-halo-2,2,6-trimethyl-4H-1,3-dioxin-4-one.

13. A process according to Claim 9 wherein the 5-halo moiety is Cl or Br.

14. The process of Claim 9 wherein the salt of the α-haloacetoacetic ester is converted to the free ester compound by treating with acid.

15. A process according to Claim 8 for the preparation of an α-haloacetoacetic ester comprising reacting a compound of formula II with a phenol compound of formula IV wherein $R^4$ is aryl and M is hydrogen at a temperature of about 100° to 200°C.

**Patentansprüche**

1. Verbindung der Formel:

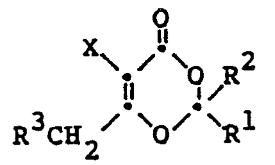

(II)

in der X für Cl oder Br steht; $R^1$ und $R^2$ jeweils unabhängig voneinander Alkyl, Aryl, substituiertes Aryl oder gemeinsam Alkylen bedeuten; und $R^3$ Wasserstoff, Cl, Br oder aliphatischer Kohlenwasserstoff, vorzugsweise von $C_1$—$C_{20}$, Alkoxy, in dem der Alkylrest $C_1$—$C_4$ ist, Aryl, substituiertes Aryl oder einen Hetero-Substituent, vorzugsweise Phenylthio, Anilino oder Diethylphosphonato darstellt.

2. Verbindung nach Anspruch 1, in dei X für Cl oder Br steht; $R^1$ und $R^2$ jeweils Methyl oder gemeinsam Tetramethylen oder Pentamethylen bedueten und $R^3$ Wasserstoff, Cl oder Br ist.

3. Verbindung nach Anspruch 1, in der X für Cl oder Br steht; $R^1$ und $R^2$ jeweils Methyl bedeuten und $R^3$ Wasserstoff ist.

4. Verfahren zur Herstellung der Verbindung von Anspruch 1, bei dem man eine Verbindung der Formel:

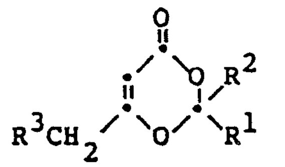

(I)

mit $X_2$ oder $SO_2X_2$ behandelt und das hierbei entstandene 5-Haloprodukt gewinnt, wobei X, $R^1$, $R^2$ und $R^3$ die in Anspruch 1 angegebene Bedeutung haben.

5. Verfahren nach Anspruch 4, in dem das Molverhältnis von $X_2$ oder $SO_2X_2$ zur Verbindung (I) etwa 1,0 bis etwa 1,2 beträgt.

6. Verfahren nach Anspruch 4, in dem man die Verbindung (I) mit elementarem Chlor oder Brom behandelt und das hier anfallende 5-Halo-Produkt gewinnt.

7. Verfahren nach Anspruch 6, in dem $R^1$ und $R^2$ jeweils Methyl bedeuten und $R^3$ für Wasserstoff, Cl oder Br steht.

8. Verfahren zur Herstellung eines α-Haloacetoessigesters der Formel:

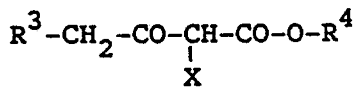

(III)

oder eines Alkalimetallsalzes seines enolischen Tautomeren, in der X für Cl oder Br steht; $R^3$ Wasserstoff, Cl, Br, aliphatischer Kohlenwasserstoff, Alkoxy, Aryl, substituiertes Aryl oder ein Hetero-Substituent ist; und $R^4$ Alkyl, Aralkyl oder Aryl bedeutet, bei dem man eine Verbindung der Formel II mit einem Reagens der Formel:

$$R^4OM \qquad (IV)$$

umsetzt, in der $R^4$ für Alkyl, Aralkyl oder Aryl steht und M ein Alkalimetallatom ist, wenn $R^4$ für Alkyl oder Aralkyl steht oder M Wasserstoff ist, wenn $R^4$ für Aryl steht.

9. Verfahren nach Anspruch 8, für die Herstellung eines Alkalimetallsalzes eines α-Haloacetoessigesters, bei dem man eine Verbindung der Formel II mit einem Alkalimetallalkoxid oder -aralkoxid der Formel IV, in der $R^4$ für Alkyl oder Aralkyl steht und M ein Alkalimetallatom ist, bei einer Temperatur von etwa −40° bis etwa 50°C umsetzt.

10. Verfahren nach Anspruch 9, in dem das Alkalimetallalkoxid Natriummethoxid oder Natriumethoxid ist.

11. Verfahren nach Anspruch 9, in dem das Molverhältnis von Alkalimetallalkoxid zu 5-Halo-4H-1,3-dioxin-4-on-Verbindung bei etwa 1,0 bis etwa 2,0 liegt.

12. Verfahren nach Anspruch 9, bei dem man ein Alkalimetallalkoxid mit 5-Halo-2,2,6-trimethyl-4H-1,3-dioxin-4-on umsetzt.

13. Verfahren nach Anspruch 9, in dem der 5-Halorest Cl oder Br ist.

14. Verfahren nach Anspruch 9, in dem das Salz des α-Haloacetoessigesters durch Behandlung mit Säure in die freie Esterverbindung überführt wird.

15. Verfahren nach Anspruch 8 für die Herstellung eines α-Haloacetoessigesters, bei dem man eine Verbindung der Formel II mit einer Phenolverbindung der Formel IV, in der $R^4$ für Aryl steht und M Wasserstoff ist, bei einer Temperatur von etwa 100° bis 200°C umsetzt.

**Revendications**

1. Composé ayant la formule:

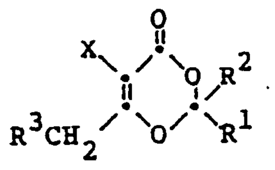

(II)

où X est Cl ou Br; $R^1$ et $R^2$ sont chacun séparément un groupe alkyle, aryle, aryle substitué ou ensemble forment un groupe alkylène; et $R^3$ est un atome d'hydrogène, de chlore ou de brome, un groupe aliphatique hydrocarboné, de préférence en $C_1$—$C_{20}$, un groupe alkoxy où le radical alkyle est de préférence en $C_1$—$C_4$, un groupe aryle, aryle substitué ou bien un hétéro substituant, de préférence phénylthio, anilino, ou diéthylphosphonato.

2. Composé selon la revendication 1, où X est Cl ou Br; $R^1$ et $R^2$ sont chacun un groupe méthyle ou ensemble un groupe tétraméthylène ou pentaméthylène; et $R^3$ est un atome d'hydrogène, de chlore ou de brome.

3. Composé selon la revendication 1, où X est Cl ou Br; $R^1$ et $R^2$ sont chacun un groupe méthyle; et $R^3$ est un atome d'hydrogène.

4. Procédé pour obtenir le composé de la revendication 1, consistant à traiter un composé de formule:

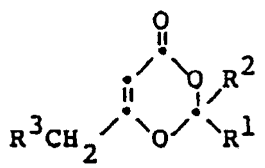

(I)

avec $X_2$ ou $SO_2X_2$ et à recueillir le produit substitué en position 5 par un atome d'halogène obtenu, où X, $R^1$, $R^2$ et $R^3$ sont tels que définis dans la revendication 1.

5. Procédé selon la revendication 4, où le rapport de $X_2$ ou $SO_2X_2$ au composé (I) est d'environ 1,0 à environ 1,2 en moles.

6. Procédé selon la revendication 44, consistant à traiter le composé (I) avec du chlore, du brome, sous forme d'élément et à recueillir le produit substitué en position 5 par un atome d'halogène obtenu.

7. Procédé selon la revendication 6, où $R^1$ et $R^2$ sont chacun un groupe méthyle; $R^3$ est un atome d'hydrogène, de chlore ou de brome.

8. Procédé de préparation d'un ester α-haloacétoacétique de formule:

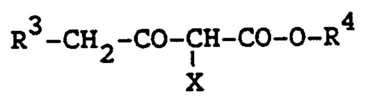

(III)

ou d'un sel de métal alcalin de son tautomère énolique, où X est Cl ou Br; $R^3$ est un atome d'hydrogène, Cl, Br, un groupe aliphatique hydrocarboné, alkoxy, aryle, aryle substitué ou un hétéro-substituant; et $R^4$ est un groupe alkyle, aralkyle ou aryle; procédé consistant à faire réagir un composé de formule II avec un réactif de formule:

$$R^4OM \qquad (IV)$$

où $R^4$ est un groupe alkyle, aralkyle ou aryle et M est un atome de métal alcalin quand $R^4$ est un groupe alkyle ou aralkyle ou M est un atome d'hydrogène quand $R^4$ est un groupe aryle.

9. Procédé selon la revendication 8, pour préparer un sel de métal alcalin d'un ester α-haloacétoacétique consistant à faire réagir un composé de formule II avec un alkoxyde ou un aralkoxyde de métal alcalin de formule IV où $R^4$ est un groupe alkyle ou aralkyle et M est un atome de métal alcalin, à une température d'environ −40°C à environ 50°C.

10. Procédé selon la revendication 9, dans lequel l'alkoxyde de métal alcalin est le méthoxyde de sodium ou l'éthoxyde de sodium.

11. Procédé selon la revendication 9, dans lequel le rapport de l'alkoxyde de métal alcalin au composé 5-halo-4H-1,3-dioxin-4-one est d'environ 1,0 à environ 2,0 en moles.

12. Procédé selon la revendication 9, dans lequel on fait réagir un alkoxyde de métal alcalin avec de la

5-halo-2,2,6-triméthyl-4H-1,3-dioxin-4-one.

13. Procédé selon la revendication 9, dans lequel le substituant halogéné en position 5 est du chlore ou du brome.

14. Procédé selon la revendication 9, dans lequel le sel de l'ester α-haloacétoacétique est transformé en ester libre par traitement avec un acide.

15. Procédé selon la revendication 8, pour préparer un ester α-haloacétoacétique consistant à faire réagir un composé de formule II avec un composé phénolique de formule IV où $R^4$ est un groupe aryle et M est un atome d'hydrogène à une température d'environ 100°C à environ 200°C.